# EUROPEAN PATENT APPLICATION

(11) **EP 4 603 469 A1**
(43) Date of publication of application: **20.08.2025**
(21) Application number: 24157530.7
(22) Date of filing: 14.02.2024
(51) Int. Cl.: C07C 5/03, C07C 29/132, C07C 29/17, C07C 51/36, C07C 41/20, C07C 35/08, C07C 33/025, C07C 43/188, C07C 62/30, C07C 62/34, C07C 57/30, C07C 57/26

(54) **METHOD FOR REDUCTION OF ORGANIC COMPOUNDS WITH MECHANOCHEMICAL TREATMENT**

(71) Applicant: Tallinn University of Technology, 19086 Tallinn (EE)
(72) Inventor: Aav, Riina, 12618 Tallinn (EE); Kananovich, Dzmitry, 12618 Tallinn (EE); Nallaparaju, Jagadeesh, 12618 Tallinn (EE)
(74) Representative: Moosedog Oy

(57) **Abstract**

A method for reduction of organic compounds, the method comprising: combining organic compounds, a solid magnesium, an amine, and an ether solvent to form a mixture; applying mechanochemical treating to the mixture to facilitate generation of solvated electrons from the solid magnesium; using solvated electrons for reduction of organic compounds followed by protonation of intermediate anionic species with the amine to obtain the reduced organic compound.

## Description

### TECHNICAL FIELD

The present disclosure relates to a method of reducing organic compounds.

### BACKGROUND

Dissolving metal-type transformations are a powerful method for reduction of organic compounds. The method is highly valuable in chemical industry because it allows to reduce even highly chemically resistant aromatic compounds. This is so-called Birch reduction, the most widely applied dissolved metal-type reduction. The Birch method for dearomatizing aromatic compounds is indispensable in the synthesis of complex natural products, pharmaceuticals, and fine chemicals from readily available aromatic feedstock. Historically, the field of dissolving metal reductions has been dominated by alkali metal reductants. For example, the classic Birch reaction transforms benzene derivatives into cyclohexadienes by reduction with alkali metals (Li, Na, K) dissolved in liquid ammonia and using alcoholic additives as a protic source. However, the use of pyrophoric alkali metals and hazardous liquid ammonia at cryogenic temperatures (below -33 °C) makes the classic approach timeconsuming, hazardous and requires tedious experimental procedure and set-up. Since the alkali metals react readily and often violently with water and oxygen in air, inert gas protection is required. Another disadvantage of the alkali metal-mediated reaction is that due to the high reactivity of the alkali metals, Birch reduction has low functional group tolerance and aromatic compounds are often over-reduced, lowering yields and adding extra steps to the purification of the target product. Other dissolving metal reductions are performed similarly and have the same shortcomings.

There have been efforts to improve the reaction's safety. For example, Benkeser's replacement of ammonia with low molecular weight amines has helped to remove liquid ammonia. Furthermore, Benkeser attempted to replace alkali metals with safer calcium. However, the robust crystalline lattice of the bulk calcium hinders its solubility and reactivity, necessitating tedious mitigation measures and makes the method unpractical. Moreover, likewise the reductions with alkali metals, the reductions with calcium are not chemoselective, giving over-reduced products of the reaction. Likewise, magnesium metal has been attempted in the Birch reduction in liquid ammonia at cryogenic temperatures but has delivered low yields of Birch reduction products and therefore is also unpractical.

Therefore, in light of the foregoing discussion, there exists a need to overcome the aforementioned drawbacks associated with the dissolving metal reduction reactions.

### SUMMARY

The aim of the present disclosure is to provide a method for dissolving metal reduction of organic compounds with bulk magnesium metal that is safer, less-time consuming, chemoselective and providing high yields of the reduced organic compounds. Advantageous features are set out in the appended dependent claims.

Embodiments of the present disclosure enable to carry out a simple and fast dissolving metal reduction reaction which is less hazardous. Low hazards are due to avoiding bulk solvents, especially liquid ammonia, and using bulk magnesium metal that does not react readily with water and air at ambient conditions. Additional advantage of the present disclosure is that the magnesium used as a sustainable replacement for lithium, an element widely used in dissolving metal reductions but with limited available reserves that is under serious threat of depletion of known resources in the coming decades. The reaction produces products with high yield and in chemoselective manner, without the need for separation of mixtures of products.

Additional aspects, advantages, features and objects of the present disclosure would be made apparent the detailed description of the illustrative embodiments construed in conjunction with the appended claims that follow.

### DETAILED DESCRIPTION OF EMBODIMENTS

The following detailed description illustrates embodiments of the present disclosure and ways in which they can be implemented.

In a first aspect, an embodiment of the present disclosure provides a method for reduction of organic compounds, the method comprising combining organic compounds, a solid magnesium, an amine, and an ether solvent to form a mixture; applying mechanochemical treating to the mixture to facilitate generation of solvated electrons from the solid magnesium and using solvated electrons for reduction of organic compounds followed by protonation of intermediate anionic species with the amine to obtain the reduced organic compound.

The method comprises combining organic compounds, a solid magnesium, an amine, and an ether solvent to form a mixture. In the method of the present disclosure, a solid magnesium is an alkaline earth metal, which is a safer alternative to commonly used hazardous sodium and potassium and offer a sustainable replacement for lithium, an element with limited available reserves (crustal abundance 0.0016%) that is under serious threat of depletion of known resources in the coming decades. On the contrary, magnesium has a crustal abundance of 2.8%. Although magnesium has lower reducing power in comparison to that of the alkali metals and calcium, its solution in ammonia can serve as strong reductant but difficult to prepare and dangerous to handle. On the other hand, the use of the bulk metal is hindered by its robust crystalline lattice and passive layer on its surface that need to be removed prior to the reaction. Moreover, generation of sparingly soluble products in the course of the reaction passivate surface of the metal and stop the reaction. Consequently, bulk magnesium is unpractical for use in dissolving metal reductions under conventional solution chemistry conditions. However, its lower reducing power is beneficial for performing chemoselective reductions. It has been shown that the use of bulk solid magnesium allows for chemoselective transformations. See Examples 2-6. Furthermore, bulk magnesium metal is safe to handle in air, since the metal does not react readily with water and air at ambient conditions.

The primary role of the amine in the method is to produce solvated electrons by dissolving solid magnesium and these solvated electrons will be used in reducing the organic compounds. The secondary role is to protonate anionic intermediates formed from organic compounds after addition of electrons. The amine also helps to stabilize reactive intermediates if such form. The specific choice of the amine can influence the outcome of the reaction. Beneficially, the amine will allow for the solid magnesium to serve as a strong reductant in the method.

The primary role of the ether solvent is to help dissolving the solid magnesium, by coordinating formed magnesium cations. The secondary role is liquid assisted grinding (LAG) additive, as the ether solvent also assist the mechanochemical treating. Ether solvents are known for their lubricative properties and therefore the ether solvent will help to reduce friction in the mixture and in turn facilitate the mechanochemical treating process, and the efficiency of the reaction. Beneficially, the ether solvents can dissolve the organic compounds and help to prevent agglomeration or clumping of the mixture during the mechanochemical treating allowing for more homogenous mixing and promoting interaction of the components due to improved mass transfer, leading to more efficient reaction. Beneficially, the ether solvents are inert towards the reaction conditions and will not undergo unwanted reactions.

The method comprises applying mechanochemical treatment to the mixture to facilitate generation of solvated electrons from the solid magnesium. The term *"mechanochemical treatment"* as used herein refers to the use of mechanical force, such as grinding, milling, shearing, or similar to induce chemical reactions in solid-state materials. This approach harnesses mechanical energy to drive chemical transformations, offering a green and efficient alternative to traditional methods that may involve solvents, high temperatures, or other harsh conditions. The mechanochemical treatment facilitates generation of solvated electrons from the solid magnesium, by revealing and constant renewal of the reactive surface of the bulk metal. The mechanochemical treatment further removes the passive layer on the surface of the metal, which otherwise prevents reactions in solution. Additionally, the mechanochemical treatment promotes the interaction of solid particles in the mixture with other components by crushing larger particles into smaller ones, thereby increasing the effective surface area of the solid particles. Additionally, the mechanochemical treatment improves mass transfer due to rapid agitation of the mixture and therefore improves the reaction rate and overall efficiency of the reaction.

The method comprises using solvated electrons for reduction of organic compounds followed by protonation of intermediate anionic species with the amine to obtain the reduced organic compound. The protonation of the intermediate anionic species directs the reaction towards the desired product, since transfer of electron can be reversible. The protonated intermediate anionic species undergo further elementary reactions to yield the final reduced organic compounds. Without the protonation, the reaction does not occur or multiple electron transfers may result in over-reduction. Furthermore, protonation controls the reduction process, allowing for the selective formation of desired isomer and only partially reduced products, without further over-reduction. Consequently, yields and chemoselectivity of the reduction process can be tuned by selecting a proper protic source.

The technical effect of the abovementioned method is that it enables reduction reactions with readily available and safer-to-handle solid magnesium, operates under air and ambient temperature conditions.

In an embodiment, the organic compounds comprise a functional group prone to reduction, wherein the functional group is selected from at least one of: arene, heteroarene, halogen, alkene, alkyne, epoxide, hydroxyl, thiol, alkoxyl, thioalkyl, carbonyl, carboxyalkyl, nitrile, imine, oxime, nitro, nitroso, sulfinyl, sulfonyl. Functional group of the organic compound determines the reactivity of the organic compound. Functional groups must be prone to reduction, meaning they should transform into other functional groups with a lower oxidation state of their atoms during the reduction process. Furthermore, chemoselectivity is observed during the reduction process. The term chemoselectivity means that the process reduces only a specific functional group in the presence of other functional groups that also prone to reduction. For example, exclusive reduction of an electron-deficient arene ring takes place in the presence of an electron-rich arene rings in the same molecule. Additional aspects of chemoselectivty include generation of a single isomer of the product among several possible isomers, or the case when the primary reduction product does not undergo further reduction (over-reduction) under the reaction conditions.

In an embodiment, reduction is a reductive cleavage. The term *"reductive cleavage"* as used herein refers to a process where an organic compound is both reduced and cleaved during the same chemical transformation. Cleavage refers to the breaking of chemical bonds. This type of reaction can lead to the formation of smaller fragments with altered oxidation states compared to the original molecule of the organic compound. Furthermore, benefit of reductive cleavage is that it is selective, allowing for cleavage of specific bonds in a molecule.

In an embodiment, the method is carried out at a temperature range from -20° C up to 100° C. The temperature may be for example from - 20, - 10, 0, 10, 20, 30, 40, 50, 60, 70, 80, 90 or 95° C up to - 15, - 5, 5, 15, 25, 35, 45, 55, 65, 75, 85 or 100° C. Beneficially, the reduction of organic compounds will already take place at room temperature and compared, no heating or cooling is needed. However, if the temperature of the mixture changes due to the changes in ambient conditions, this does not influence the results. Furthermore, for some mixtures, variation in the temperature can be used to control the reaction efficiency, selectivity and yield.

In an embodiment, an amine is selected from a group comprising at least one of: primary alkyl amines, secondary alkyl amines, primary cycloalkyl amines, secondary cycloalkyl amines, heterocyclic secondary amines containing at least 2 nitrogen atoms, and N-alkylated derivatives thereof. The amines used for the method of reducing organic compounds are amines that facilitate generation of solvated electrons, by chelating metal cation and generating putative metal electrides. The amines also possess weak Brønsted acidity so that they can protonate the intermediate anionic species. This step is crucial for stabilizing the intermediate anionic species and directing the reaction towards the reduced organic compounds. The function of the amine is to function as a protic source without participating in nucleophilic reactions with other compounds in the mixture.

Beneficially, these amines are stable in the presence of the solid magnesium, do not undergo reduction or reductive cleavage themselves. Stability is essential to prevent unwanted reactions between the amine, organic compound and the solid magnesium, ensuring the integrity of the reaction conditions.

In an embodiment, the ether solvent is selected from a group comprising at least one of: THF, 2-MeTHF, tetrahydropyran. THF is the most commonly used ether solvent due to its high affinity to magnesium cations, good chemical resistance in a reductive cleavage reaction, and compatibility with a wide range of organic compounds. Relatively low boiling point of THF makes is suitable for low-temperature conditions or when there is need to remove THF from the mixture. 2-MeTHF is suitable for higher temperatures due to the higher boiling point than THF. Beneficially, 2-MeTHF is considered more environmentally friendly than THF, making it a preferable alternative for THF. The advantage of tetrahydropyran is that is exhibits higher chemical resistance and is beneficial when there is possibility of moisture in the mixture. Due to the lower reactivity tetrahydropyran is less prone to side reactions.

In an embodiment, the mechanochemical treating is carried out using at least one of selected from a shaker ball mill, planetary ball mill, attritor ball mill, tumbling ball mill, pestle and mortar, mechanical press, twin screw extruder, resonant acoustic mixer. The most common apparatus for the mechanochemical treating is a shaker ball mill which is simple and easy to operate and can be used for different sample sizes. Planetary ball mill allows high-energy milling due to the planetary motion and therefore provides efficient mixing and uniform grinding. Furthermore, the planetary ball mill provides good control over milling parameters. The attritor ball mill allows high-energy milling with the use of agitated balls, beneficially being efficient for fine and ultrafine grinding with providing control over particle size distribution. The tumbling ball mill is with a simple design and operation and is suitable for large-scale production. Using pestle and mortar is a simple and traditional method which is suitable for small-scale reactions and provides control over grinding intensity. The mechanical press is suitable for the mechanical treating that needs higher pressure and is suitable for powdered materials. The twin screw extruder is suitable for larger-scale production and allows to control the feeding and mixing process. The resonant acoustic mixer is a non-contact mixing method that is suitable for delicate or heat-sensitive mixtures allowing precise control over mixing intensity. The use of different apparatuses for the mechanical treating allows to choose the mechanical treating that is available and most suitable for the application.

In an embodiment, the shaker mill frequency is in a range from 3 Hz up to 80 Hz. The shaker mill frequency may be for example from 3, 5, 15, 25, 35, 45, 55, 65 or 75 Hz. up to 5, 10, 20, 30, 40, 50, 60, 70, or 80 Hz. The shaker mill frequency will have an influence on the efficiency, yield and selectivity of the reduction of the organic compounds. The frequency must be optimal to ensure fast agitation of the reaction mixture and activation of the surface of the bulk metal through ball impacts. However, if the frequency is too high, there could be side reactions promoted, resulting in a lower yield. Higher frequencies can also generate excessive friction heat that will impact the temperature of the mixture. Lower frequencies can be used to control the reduction reaction pathways as well as the temperature and therefore will allow for better efficiency and selectivity.

In an embodiment, the mechanochemical treating is carried out from at least 5 minutes up to 12 h. The effect of mechanochemical treating depends on how it is carried out. By varying the mechanochemical treating duration, reaction kinetics, particle size, surface area, activation energy, temperature control and product selectivity can be controlled. When the mechanochemical treating is shorter, there is less occurrence of side reactions or undesired over-reduction by-products, meaning that the method has better chemoselectivity. When the mechanochemical treating is longer, the solid magnesium surface area is increased and in turn the reaction will be more efficient and have higher yields. Longer period will also allow to proceed with the reaction at lower temperatures.

In an embodiment, the mixture comprises the solid magnesium in a range of 3 equiv. up to 30 equiv., the amine in a range of 1 equiv. up to 10 equiv., and the ether solvent in a range of 1 equiv. up to 10 equiv. The term *"equiv."* as used herein refers equivalent where equiv. is a unit of measure used to express the amount of a substance in a chemical reaction, specifically in relation to the amount of another substance involved in the reaction. One equiv. of a substance is the amount that reacts with or is equivalent to one mole of another substance in a chemical reaction. The solid magnesium may be for example from 3, 10, 15, 20, 25, or 25 equiv. up to 5, 12, 17, 22, 27 or 30 equiv. The amine may be for example from 1, 2, 4, 6, 8 or 9.5 equiv. up to 3, 5, 7 or 10 equiv. The technical effect of having the solid magnesium in an excess is that then the surface area of the solid magnesium is higher and the reaction will have higher efficiency and yield. The excess the amine ensures the availability of sufficient solvated electrons for reduction and sufficient protons to stabilize the intermediate anionic species and promote the desired reduction. This stabilization is important for the intermediate anionic species to survive and participate in subsequent steps of the reaction without undergoing undesirable side reactions. By providing an excess of the amine, the kinetics of the reaction can be tuned, side-processes are attenuated, and the reaction can be controlled. Beneficially, the excess of the amine allows to maintain a uniform and well-defined environment of the mixture, preventing localized variations in the solvated electrons and enhancing the selectivity and efficiency of the reduction. The ether solvent may be for example from 1, 2, 4, 6, 8 or 9.5 equiv. up to 3, 5, 7 or 10 equiv. The excess of the ether solvent can be beneficial for suppressing the side reactions, providing higher yields and purer products.

In an embodiment, a method further comprises adding a protic source to the mixture. The term *"protic source"* as used herein refers to a substance that can donate protons meaning that they have hydrogen atoms capable of ionizing as protons. Although the amine also acts as the protic source, adding another protic source to the mixture can be used to improve the selectivity and yield of the method of reducing organic compounds because it facilitates protonation of intermediate anionic species.

In an embodiment, the protic source is weak solid or liquid organic or inorganic Brønsted acid. Brønsted acids are substances that are defined based on their involvement in proton transfer reactions and are defined by the broader range of solvents and reaction environments in which proton transfer reactions can occur. Beneficially, since Brønsted acids can participate in reactions in non-aqueous solvent, these are suitable for the method of reducing organic compounds which does not contain water. Furthermore, the weak Brønsted acids are preferred because these allow for better control over the extent of proton transfer in a reaction. Weak Brønsted acid provides a controlled and gradual release of protons, allowing for more nuanced adjustments in reaction conditions, allowing to avoid unwanted side reactions and allow better selectivity of the method. From the safety point of view, the weak Brønsted acids are generally less corrosive and less aggressive than strong Brønsted acids and this property is advantageous in handling and safety considerations, especially in laboratory and industrial setting.

Because the mechanochemical treating is applied, solid organic and inorganic protic sources can be used. The advantage of using weak solid organic and inorganic Brønsted acids serves as an aid for the mechanochemical treatment and help to maintain the powder-like appearance of the mixture. The advantages of using weak liquid organic and inorganic Brønsted acids include enhancing the reactivity of the mixture by improving mass transfer, promoting intimate mixing and controlling the temperature by absorbing and dissipating heat generated in the mixture when mechanochemical treating is applied.

In an embodiment, the protic source is selected from solid salts comprising at least one of selected from ammonium chloride, ammonium sulfate, ammonium hydrogen phosphate, ammonium dihydrogen phosphate, organic amine hydrochloride, organic amine sulphate. These are affordable, non-flammable, and easily-to-handle solids. Beneficially, the protic source that is a solid salt, can be used for the mechanochemical treating serving as a grinding aid and maintains powder-like appearance of the reaction mixture. Furthermore, these outpace liquid additives in term of yields and product selectivity.

In an embodiment, the protic source is selected from a group comprising at least one of: alcohols, primary amines or secondary amines comprising methanol, ethanol, 2-propanol, tert-butanol, 1-butanol, 2-butanol, ethylene glycol, glycerol, propylamine, isopropylamine, butylamine. Such protic sources are sources that are in the liquid form. Due to being in the liquid form these protic sources allow for more uniform distribution of protons throughout the mixture, which in turn allow better controlled reactions in the mixture, preventing localized reactions in the mixture. Beneficially, the reaction is more homogeneous and the reaction can take place in multiple parts of the mixture. Furthermore, this allows higher efficiency, chemoselectivity and yields. More acidic alcohols (such as methanol and ethanol) may give faster reductions but may deliver lower yields than bulkier alcohols (such as isopropanol) and therefore by varying the protic source, a desired outcome can be achieved by choosing the protic source.

In an embodiment, the solid magnesium is selected from at least one of the following forms: powder, chips, turnings, foil, beads or granules.

Depending on the industrial applications or the origin of the solid magnesium, it can in different forms. The term *"powder"* as used herein refers to the solid magnesium that consists of fine particles of the solid magnesium. The solid magnesium in form of a powder has good reactivity due to the high surface are but compared to other solid magnesium forms is slightly more reactive. The term *"chips"* as used herein refers to the solid magnesium that is in small pieces or shavings and is suitable because of generation high surface area metal particles during mechanochemical treatment. The term *"turnings"* as used herein refers to the solid magnesium that is similar to the chips but is typically longer and more twisted in shape. Similarly to the chips, turnings generate high surface area metal particles during mechanochemical treatment for the enhanced reactivity. The term *"foil"* as used herein refers to the solid magnesium that is a thin sheet of solid magnesium and is beneficial for generating high surface area metal particles during mechanochemical treatment. The term *"beads"* as used herein refers to the solid magnesium that is made of small, spherical-shaped forms and are also suitable for chemical reactions, in applications where slower reactivity of the solid magnesium is desired due to the smaller surface area of individual bead particle and recovery of the excess of metal is required. The term *"granules"* as used herein refers to the solid magnesium that are made of small, but irregularly shaped forms. These can be used for controlled reaction conditions because granules provide a compromise between the high surface are of the powder and the reduced reactivity of the larger pieces. The technical effect of different forms of the solid magnesium is that specific form will influence the selectivity, efficiency and yield of the reaction.

### EXAMPLES

### Example 1. Birch reduction of 3-fluorobenzoic acid.

A 15 mL milling jar was loaded under air with 3-fluorobenzoic acid (200 mg, 1.42 mmol), magnesium powder (346 mg, 14.2 mmol, 10 equiv.), ethylenediamine (429 mg, 7.13 mmol, 5 equiv.), THF (116 µL, 1 equiv.). A single 10 mm milling ball (weight ca. 3 g) was added, and the mixture was milled at 30 Hz for 60 minutes at room temperature. After quenching with aqueous hydrochloric acid, the analysis showed the formation of 3-fluorocyclohexa-2,5-diene-1-carboxylic acid in 91% yield. Importantly, only less than 5% of cyclohexa-2,5-diene-1-carboxylic acid is formed as a result of a competitive reductive defluorination process. On the contrary, the same reaction with more reactive calcium metal affords only 52% yield of 3-fluorocyclohexa-2,5-diene-1-carboxylic acid due to enhanced reductive defluorination process.

### Example 2. Birch reduction of 3-(4-methoxybenzyl)benzoic acid.

A 15 mL milling jar was loaded under air with 3-(4-methoxybenzyl)benzoic acid (100 mg, 0.41 mmol), magnesium (100 mg, 4.11 mmol, 10 equiv.), ethylenediamine (124 mg, 2.06 mmol, 5 equiv.), THF (34 µL, 1 equiv.). A single 10 mm milling ball (weight ca. 3 g) was added, and the mixture was milled at 30 Hz for 60 minutes at room temperature. After quenching with aqueous hydrochloric acid, the analysis showed the formation of formation of 3-(4-methoxybenzyl)cyclohexa-2,5-diene-1-carboxylic acid in 79% yield. Only electron-deficient benzene ring was reduced. On the contrary, the same reaction with more reactive calcium metal affords this compound in only 20% yield, in a mixture with other reduction products.

### Example 3. Selectivity studies in the Birch reduction of arenes.

A 15 mL milling jar was loaded under air with benzoic acid (**1a**, 122 mg, 1 mmol), n-BuOPh (**2a**, 150 mg, 1 mmol), metal (Li: 24 mg, 3.5 mmol, 3.5 equiv; Ca: 80 mg, 2 mmol, 2 equiv.; Mg: 242 mg, 9.95 mmol, 10 equiv.), ethylenediamine (300 mg, 4.99 mmol, 5 equiv.) and THF (81 µL, 1 equiv.). A single 10 mm milling ball (weight ca. 3 g) was added, and the mixture was milled at 30 Hz for 60 minutes at room temperature.

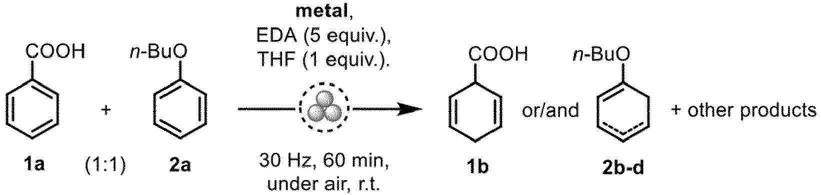

Table 1 shows yields determined by ¹H NMR in CDCl₃ using cyclododecane as an internal standard. Calcium and lithium result in Birch reduction of both electron-deficient **1a** and electron-rich **2a.** On the contrary, magnesium reduces exclusively electron-poor compound **1a.** For calcium, only trace of **1b** was observed, though significant amount of **1a** was consumed. HPLC-MS analysis showed the formation of unidentified products (see below), which could arise from some intramolecular reactions between **1a, 2a** and the respective reduction products.

**Table 1.**

| **Metal (equiv)** | **¹H NMR yields (%)** | | | |
|---|---|---|---|---|
| | **1a** | **1b** | **2a** | **2b-d** |
| Lithium (3.5 equiv) | 62 | 21 | 53 | 30 |
| Calcium (2 equiv) | 58 | 1 | 17 | 77 |
| Magnesium (10 equiv) | 12 | 59 | 92 | 0 |

Table 2 shows data obtained from the HPLC analysis which shows that with using magnesium, only **1a** was reduced and **2a** remained in the reaction mixture without being reduced.

**Table 2.**

| **Metal (equiv)** | **ratio of starting materials (1a:2a) remained in the reaction mixtures** |
|---|---|
| | **1a:2a** |
| Lithium (3.5 equiv) | 44:56 |
| Calcium (2 equiv) | 79:21 |
| Magnesium (10 equiv) | 5:95 |

### Example 4. Reduction of (E)-1,2-diphenylethene.

A 15 mL milling jar was loaded with (*E*)-1,2-diphenylethene (200 mg, 1.10 mmol), magnesium (270 mg, 11.1 mmol, 10 equiv.), ethylenediamine (333 mg, 5.54 mmol, 5 equiv.) and THF (90 µL, 1 equiv.). A single 10 mm milling ball (weight ca. 3 g) was added, and the mixture was milled at 30 Hz for 60 min at room temperature. After quenching with aqueous hydrochloric acid, the analysis showed the formation of 1,2-diphenylethane as a single product in 98% yield. By contrast, the same reduction with calcium results in only 48% yield of 1,2-diphenylethane, due to generation of by-products with partially reduced aromatic rings.

### Example 5. Reduction of hex-5-yn-1-ol.

A 15 mL milling jar was loaded with hex-5-yn-1-ol (200 mg, 2.03 mmol,), magnesium (495 mg, 20.3 mmol, 10 equiv.), ethylenediamine (612 mg, 10.1 mmol, 5 equiv.), and THF (165 µL, 1 equiv.). A single 10 mm milling ball (weight ca. 3 g) was added, and the mixture was milled at 30 Hz for 60 min at room temperature. After quenching with aqueous hydrochloric acid, the analysis showed the formation of hex-5-en-1-ol as a single product in 40% yield and 60% of the starting material was recovered. By contrast, the same reduction with calcium results in only 15% yield of hex-5-en-1-ol, as 85% of the product is over-reduced to hexan-1-ol.

### Example 6. Reduction of cinnamic acid.

A 15 mL milling jar was loaded with cinnamic acid (200 mg, 1.34 mmol), magnesium (328 mg, 13.4 mmol, 10 equiv.), ethylenediamine (405 mg, 6.74 mmol, 5 equiv.) and THF (110 µL, 1 equiv.). A single 10 mm milling ball (weight ca. 3 g) was added, and the mixture was milled at 30 Hz for 60 min at room temperature. After quenching with aqueous hydrochloric acid, the analysis showed the formation of hydrocinnamic acid as a single product in 95% yield. By contrast, the same reduction with calcium results in over-reduction of the aromatic ring.

### Example 7. Reduction of epoxycyclohexane.

A 15 mL milling jar was loaded with epoxycyclohexane (200 mg, 2.03 mmol), magnesium (495 mg, 20.3 mmol, 10 equiv.), ethylenediamine (612 mg, 10.2 mmol, 5 equiv.), and THF (165 µL, 1.0 equiv.). A single 10 mm milling ball (weight ca. 3 g) was added, and the mixture was milled at 30 Hz for 90 min at room temperature. After quenching with aqueous hydrochloric acid, the analysis showed the formation of cyclohexanol as a single product in 95% yield.

## Claims

1. A method for reduction of organic compounds, the method comprising:
- combining organic compounds, a solid magnesium, an amine, and an ether solvent to form a mixture;
- applying mechanochemical treating to the mixture to facilitate generation of solvated electrons from the solid magnesium;
- using solvated electrons for reduction of organic compounds followed by protonation of intermediate anionic species with the amine to obtain the reduced organic compound.

2. A method according to claim 1, wherein the organic compounds comprise a functional group prone to chemoselective reduction, wherein the functional group is selected from at least one of: arene, heteroarene, halogen, alkene, alkyne, epoxide, hydroxyl, thiol, alkoxyl, thioalkyl, carbonyl, carboxyalkyl, nitrile, imine, oxime, nitro, nitroso, sulfinyl, sulfonyl.

3. A method according to claims 1 or 2, wherein reduction is a reductive cleavage.

4. A method according to any of the preceding claims, wherein the method is carried out at a temperature range from -20° C up to 100° C.

5. A method according to any of the preceding claims, wherein an amine is selected from a group comprising at least one of: primary alkyl amines, secondary alkyl amines, primary cycloalkyl amines, secondary cycloalkyl amines, heterocyclic secondary amines containing at least 2 nitrogen atoms, and N-alkylated derivatives thereof.

6. A method according to any of the preceding claims, wherein the ether solvent is selected from a group comprising at least one of: THF, 2-MeTHF, tetrahydropyran.

7. A method according to any of the preceding claims, wherein the mechanochemical treating is carried out using at least one of selected from a shaker ball mill, planetary ball mill, attritor ball mill, tumbling ball mill, pestle and mortar, mechanical press, twin screw extruder, resonant acoustic mixer.

8. A method according to claim 8, wherein the shaker mill frequency is in a range from 3 Hz up to 80 Hz.

9. A method according to any of the preceding claims, wherein the mechanochemical treating is carried out from at least 5 minutes up to 12 h.

10. A method according to any of the preceding claims, wherein the mixture comprises
- the solid magnesium in a range of 3 equiv. up to 30 equiv.,
- the amine in a range of 1 equiv. up to 10 equiv.,
- the ether solvent in a range of 1 equiv. up to 10 equiv.

11. A method according to any of the preceding claims, further comprising adding a protic source to the mixture.

12. A method according to claim 11, wherein the protic source is weak solid or liquid organic or inorganic Brønsted acids.

13. A method according to claim 11, wherein the protic source is selected from solid salts comprising at least one of selected from ammonium chloride, ammonium sulfate, ammonium hydrogen phosphate, ammonium dihydrogen phosphate, organic amine hydrochloride, organic amine sulphate.

14. A method according to claim 11, wherein the protic source is selected from a group comprising at least one of: alcohols, primary amines or secondary amines comprising methanol, ethanol, 2-propanol, tert-butanol, 1-butanol, 2-butanol, ethylene glycol, glycerol, propylamine, isopropylamine, butylamine.

15. A method according to any of the preceding claims, wherein the solid magnesium is selected from at least one of the following forms: powder, chips, turnings, foil, beads or granules.
